# EUROPEAN PATENT APPLICATION

(11) **EP 3 797 833 A1**
(43) Date of publication of application: **31.03.2021**
(21) Application number: 19807512.9
(22) Date of filing: 21.05.2019
(51) Int. Cl.: A61N 7/00

(54) **GOUT AND PAIN TREATMENT DEVICE**

(30) Priority: 21.05.2018 CN 201810486466
(71) Applicant: Shanghai Sixth People's Hospital, Shanghai 200233 (CN)
(72) Inventor: ZHENG, Yuanyi, Shanghai 200233 (CN); HU, Bing, Shanghai 200233 (CN); CAI, Xiaojun, Shanghai 200233 (CN)
(74) Representative: Cabinet Laurent & Charras
(86) International application number: PCT/CN2019/087846
(87) International publication number: WO 2019/223700

(57) **Abstract**

Disclosed is an ultrasonic treatment device, comprising a main machine (1) and a therapeutic patch (2). The main machine comprises a power source (101), a main controller (102), an input panel (103), and an ultrasonic generator (104). The therapeutic patch comprises a substrate (201) and an ultrasonic probe (202). The main controller is connected to the power source, the input panel, and the ultrasonic generator, respectively. The ultrasonic generator is connected to the ultrasonic probe, and the ultrasonic probe is attached to the substrate. One or a plurality of therapeutic patches are present, and each therapeutic patch contains one or more ultrasonic probes. When a plurality of ultrasonic probes are present on a single therapeutic patch, the ultrasonic probes are arranged in an array on the substrate. The device achieves simple and convenient, long term treatment for gout at various joints or sites of the human body without side effects, and alleviates the pain of patients.

## Description

### Technical Field

The present invention relates to the field of ultrasonic therapy, in particular to a wearable ultrasonic treatment device for treating gout and alleviating the pain.

### Background

Gout is a crystal-associated arthropathy caused by monosodium urate (MSU) deposition, and is directly related to hyperuricemia caused by purine metabolism disorders, or/and hyperuricemia caused by decreased uric acid excretion .

There are many symptoms of gout, the most common of which is acute gouty arthritis, and the typical attack is characterized by sudden red, swollen and heat pain in the involved joints, and the most common involved joints are the first metatarsophalangeal joints, and it can also involve the ankle, knee and tarsal joints. The gout may be relieved in several days and may attack periodically. Those who suffer from a long history and repeated attacks of the gout may have tophus deposition, joint destruction, loss of function, and disappearance of diapause, and the disease (the gout) may developed into chronic tophaceous gout. The disease may further lead to urate nephropathy and renal insufficiency, posing a severe threat to patients' health.

The current gout therapy is to control the level of uric acid, which requires long-term or even lifelong medication, and does not treat the gout disease radically. The following problems exist on either western medicine or traditional Chinese medicine: first, the severe toxic side effects; second, the fragile patient compliance; not least, the high cost of medication, which brings a heavier financial burden to patients.

It will be beneficial to patients' both physical and mental health if there is a safe and effective physiotherapy which can be used instead of medication. In addition, since modern people lead a stressful life and thereby their time is precious, it is too time-consuming to commute to the hospital and spend time waiting for medical diagnosis. As for those critical patients with inconvenience, it would be a still worse choice to go to the hospital chronically. Therefore, it is desirable to provide a compact , easy-to-use , portable and handy gout treatment device and treatment method thereof, for patients who can treat themselves according to doctors' instructions at any time.

In the prior art, there are energy-based treatments for gout such as U.S. patent publication No. US9597099 which discloses a method of performing a medical procedure. The method comprises of placing an energy emitting device near a uric acid crystal with a certain resonant frequency, exerting energy of a predetermined frequency to the tissue by using the energy emitting device, and thereby causing the uric acid crystal to disintegrate thoroughly or partly, and then moving out the disintegrated uric acid. However, this patent does not demonstrate the specific form of energy used to disintegrate the uric acid crystals, neither does it mention the effect it may achieve.

Besides, some prior art discloses portable or wearable treatment devices, as is mentioned in CN103977507 and CN2527303. Admittedly, the devices disclosed have significantly reduced in terms of overall size and area of the treatment compared to large or medium-sized physiotherapy devices used in medical institutions, and the problem of portability has been solved, nevertheless, they are not convenient for long-term use, especially for joints with irregular shapes that vary from person to person. Thus, it is difficult for effective use of the devices in the treatment of gout or other body and joint pains.

### Summary

To overcome the deficiencies of the prior art, the present invention aims to provide a treatment device and treatment method for gout and pain alleviation, which can be simple and convenient to operate for long term-use, and can be widely applied to various joints or parts of the human body without side effects.

In order to solve the aforementioned technical problem, the technical solution of the present invention is that: An ultrasonic treatment device comprising a main machine (1) and a therapeutic patch (2), the main machine comprises a power source (101), a main controller (102), an input panel (103), and an ultrasonic generator (104), the therapeutic patch comprises a substrate (201) and an ultrasonic probe (202); the main controller is connected to the power source, the input panel, and the ultrasonic generator, respectively, the ultrasonic generator is connected to the ultrasonic probe, and the ultrasonic probe is attached to the substrate; wherein, one or more therapeutic patches are present, and each therapeutic patch contains one or more ultrasonic probes; when a plurality of ultrasonic probes are present on a single therapeutic patch, the ultrasonic probes are arranged in an array on the substrate.

In another preferred embodiment, the size of and the thickness of the ultrasonic probe are 4mm²-8cm² and ≤ 10mm², respectively.

More preferably, the size of the ultrasonic probe is 4mm²-2mm².

More preferably, the shape of the ultrasonic probe is circular.

In another preferred embodiment, the power and frequency of the ultrasonic waves transformed by the ultrasonic probe are 0.5-5w/cm² and 200KHz-5MHz, respectively.

In another preferred embodiment, the ultrasonic generator is in intermittent or continuous mode, and the intermittent ultrasound emission mode has an intermittent time of 100-10,000 ms.

In another preferred embodiment, the substrate has a fix portion that is adhesive or absorbent.

In another preferred embodiment, the power supply is a DC battery, a rechargeable battery or an external power converter, with a voltage of ≤20V.

In another preferred embodiment, the therapeutic patch further comprises a temperature sensor, the temperature sensor is connected to and is controlled by the main controller, and feeds back the temperature signals to the main controller.

In another preferred embodiment, the main machine and the therapeutic patch are integrated as a whole.

The beneficial effect of the present invention is that, the present invention can realize the effective alleviation or treatment of gout or pain by ultrasound, especially, for joints with irregular shapes that vary from person to person, it is universally applicable. It can be carried around and used anytime and anywhere according to the patient's need, and has side effects, convenient, safe and effective without side effects.

### Brief Description of Drawings

FIG. 1 is a schematic diagram of the gout and pain treatment device of the present invention;
FIG. 2 is a schematic diagram of the gout and pain treatment device according to one of the embodiments of the present invention;
FIG. 3 is a schematic diagram of the appearance of the gout and pain treatment device according to one of the embodiments of the present invention.
FIG. 4 is a picture of appearance of the gout and pain treatment device according to one of the embodiments of the present invention.
FIG. 5 shows the comparison of two states of a fresh tophus specimen taken out from the human body, respectively before and after irradiation with the treatment device of the present invention.
FIG. 6 indicates the comparison of two states of another fresh tophus specimen removed from the human body, respectively before and after irradiation with the treatment device of the present invention.

Reference signs of components in the drawings are as follows: main machine (1), therapeutic patch (2), power source (101), main controller (102), input panel (103), ultrasonic generator (104), substrate (201), ultrasonic probe (202), temperature sensor (203); A- state of the tophus before ultrasonic irradiation, B- state of the tophus after 30 minutes of ultrasonic irradiation, C- state of the tophus after irradiation.

### Embodiments

Preferred embodiments of the invention are described in detail below in combination with the drawings so that the advantages and features of the invention can be better understood by the skilled in the art, and therefore, the range of protection of the invention can be more clearly defined.

### Example 1

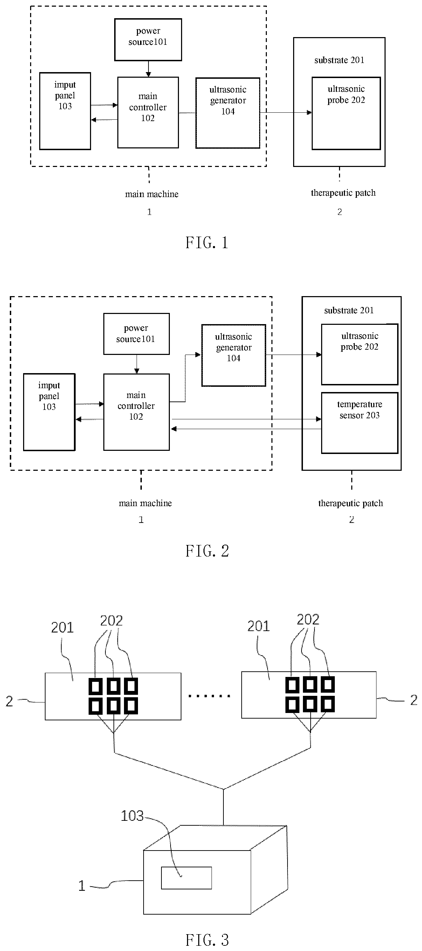

Referring to FIG. 2, the present invention is a gout and pain treatment device comprising a main machine (1) and a therapeutic patch (2), wherein the main machine comprising a power supply (101), a main controller (102), an input panel (103) and an ultrasonic generator (104), and the therapeutic patch comprising a substrate (201), an ultrasonic probe (202) and a temperature sensor (203).
The power supply (101) is a DC battery, a rechargeable battery or an external power converter, with a voltage of ≤20V.
The main controller (102) comprises a microprocessor, the role of which is to control the signal output of the ultrasound module in the treatment unit, and cooperating with the sensed feedback which is sensed by the temperature sensor according to the treatment parameters set by the user, within the technical specifications of the device, so that the whole device works stably and reliably.

The input panel (103) is a window for user interaction with the machine, and the input panel (103) comprises buttons, keypads or a touch screen for setting various parameters of the machine, and a display, a display light or a display screen for showing the operating status of the machine.

The ultrasonic generator (104) produces ultrasonic waves, the power and frequency of the produced ultrasonic waves are 0.5-5w/cm² and 200KHz-5MHz, respectively, and the frequency is adjustable according to different parts of the human body.

One or more therapeutic patches (2) is/are present, and the plurality of therapeutic patches may be freely arranged or combined to form different shapes when in use; each therapeutic patch contains one or more ultrasonic probes, and when a plurality of ultrasonic probes are present on a single therapeutic patch, the ultrasonic probes are arranged in an array on the substrate.

The substrate (201) is used for attaching the therapeutic patch to a joint or part of the body and securing it with an fix portion that is adhesive or absorbent.

The ultrasonic probe (202) is the core functional area of the device, when the therapeutic patch is in contact with the patient's joints or other painful parts, the ultrasonic probe conducts ultrasound to the tissues where gout hits, so that the depositions such as uric acid crystals or tophus in the tissues are disintegrated thoroughly or partly under vibration; the ultrasonic probe may be of regular or irregular shape, one or more in number, with a thickness of ≤ 10 mm, a size of 4 mm² to 8 cm², and ultrasound emission mode is intermittent or continuous, where the intermittent ultrasound emission mode has an intermittent time of 100-100,000 ms; during the treatment, the ultrasonic probe has a slight heating function.

The temperature sensor (203) comprises a temperature sampling circuit that monitors the temperature via a thermal element and feeds it (temperature) back to the main controller, which controls it(the temperature sampling circuit) based on preset parameters of the user or on safe operating parameters of the device.

### Example 2

Referring to FIG. 3, the present invention is a gout and pain treatment device comprising a mainframe (1) and a therapeutic patch (2). The main machine and the therapeutic patch are connected by a wire, and the main machine comprises a power supply, a main controller, an input panel (103), and an ultrasonic generator. The main machine is encased in a housing, and the power supply, the main controller and the ultrasonic generator are all located in the housing. Low-voltage rechargeable batteries are used as the power supply. Each therapeutic patch contains a plurality of ultrasonic probes (202), which are attached to a substrate (201). In this example a plurality of therapeutic patches are present, and each group (of therapeutic patches) can be applied separately to the body part where the gout occurs according to the therapeutic needs, and a plurality of therapeutic patches can also be combined and applied to the same body part, and the substrate can be rubberized fabric of medical-use.

### Example 3

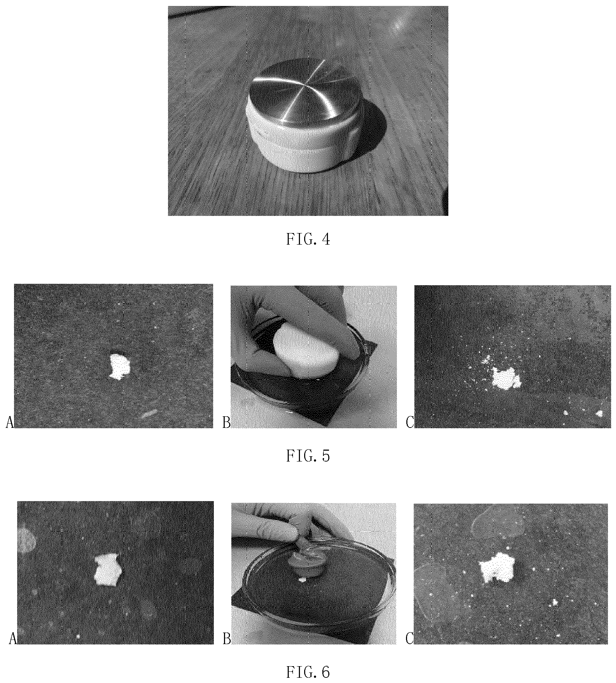

Referring to FIG.4, the present invention is a gout and pain treatment device comprising a machine and a therapeutic patch. The main machine and the therapeutic patch are integrated as a whole. The therapeutic patch contains an ultrasonic probe, and the ultrasonic probe is circular in shape.

### Example 4

In this example, a gout and pain treatment device of the present invention is used to irradiate a fresh tophus specimen removed from the human body. The test method is: place the fresh tophus specimen from the human body in a culture dish, add a small amount of liquid (saline) to the culture dish, have the liquid submerge the tophus specimen, and the role of the liquid is to simulate the environment in human tissue for the tophus. Make the ultrasonic probe (the part in contact with the irradiated object is a disk of 3cm in diameter) of the treatment device in contact with the liquid, turn on the ultrasonic treatment device, set the ultrasonic frequency to 800KHz, and intensity to 2.5w/cm², and irradiation time to 30 minutes. The tophus appears to partly collapse and dissolve after irradiation. Refer to FIG.5 in which the two states of a tophus specimen respectively before and after irradiation are shown. These results indicate that the treatment device and method of the present invention is effective in the treatment of gout.

### Example 5

In this example, a gout and pain treatment device of the present invention is used to irradiate a fresh tophus specimen removed from the human body. The test method is: place the fresh tophus specimen from the human body in a culture dish, add a small amount of liquid (saline) to the culture dish, have the liquid submerge the tophus specimen, and the role of the liquid is to simulate the environment in human tissue for the tophus. Make the ultrasonic probe (the part in contact with the irradiated object is a disk of 3cm in diameter) of the treatment device in contact with the liquid, turn on the ultrasonic treatment device, set the ultrasonic frequency to 800KHz, and intensity to 2w/cm2, and irradiation time to 30 minutes. The tophus appears to partly collapse and dissolve after irradiation. Refer to FIG.6 in which the two states of a tophus specimen respectively before and after irradiation are shown. These results indicate that the treatment device and method of the present invention is effective in the treatment of gout.

The aforementioned are only examples of the present invention and are not intended to limit the range of the patent protection of the present invention. Any equivalent structure or equivalent process transformation made by using the contents of the specification and drawings of the present invention or applied directly or indirectly in other related technical fields is similarly included in the range of patent protection of the present invention.

## Claims

1. An ultrasonic treatment device comprising a main machine (1) and a therapeutic patch (2), the main machine comprises a power source (101), a main controller (102), an input panel (103), and an ultrasonic generator (104), the therapeutic patch comprises a substrate (201) and an ultrasonic probe (202); the main controller is connected to the power source, the input panel, and the ultrasonic generator, respectively, the ultrasonic generator is connected to the ultrasonic probe, and the ultrasonic probe is attached to the substrate; wherein, one or more therapeutic patches are present, and each therapeutic patch contains one or more ultrasonic probes; When there are plurality of ultrasound probes for a individual treatment patch, the ultrasound probes are arranged in an array on the substrate.

2. The ultrasonic treatment device according to claim 1, wherein the size of and the thickness of the ultrasonic probe are 4mm²-8cm² and ≤10mm², respectively.

3. The ultrasonic treatment device according to claim 2, wherein the size of the ultrasonic probe is 4mm²-2mm².

4. The ultrasonic treatment device according to claim 2, wherein the shape of the ultrasonic probe is circular.

5. The ultrasonic treatment device according to claim 1, wherein the power and frequency of the ultrasonic waves transformed by the ultrasonic probe are 0.5-5w/cm² and 200KHz-5MHz, respectively.

6. The ultrasonic treatment device according to claim 1,wherein the ultrasonic generator is in intermittent or continuous mode, and the intermittent ultrasound emission mode has an intermittent time of 100-100,000 ms.

7. The ultrasonic treatment device according to claim 1, wherein the material of the substrate is flexible material, the substrate has a fix portion that is adhesive or absorbent.

8. The ultrasonic treatment device according to claim 1, wherein the power supply is a DC battery, a rechargeable battery or an external power converter, with a voltage of ≤20V.

9. The ultrasonic treatment device according to claim 1, wherein the therapeutic patch further comprises a temperature sensor, the temperature sensor is connected to and is controlled by the main controller, and feeds back the temperature signals to the main controller.

10. The ultrasonic treatment device according to claim 1, wherein the main machine and the therapeutic patch are integrated as a whole.

11. The ultrasonic treatment device according to any one of claims 1-10, wherein it is used for treating gout and alleviating the pain.
